# EUROPÄISCHE PATENTANMELDUNG

(11) **EP 4 197 619 A1**
(43) Veröffentlichungstag der Anmeldung: **21.06.2023**
(21) Anmeldenummer: 22214105.3
(22) Anmeldetag: 16.12.2022
(51) Int. Cl.: B01D 53/22, C01B 3/50

(54) **VERFAHREN ZUR HERSTELLUNG VON KOHLENMONOXID-HALTIGEN STRÖMEN**

(30) Priorität: 20.12.2021 EP 21215852
(71) Anmelder: Evonik Operations GmbH, 45128 Essen (DE)
(72) Erfinder: Franke, Robert, 45772 Marl (DE); Kreis, Peter, 44227 Dortmund (DE); Fridag, Dirk, 45721 Haltern am See (DE)
(74) Vertreter: Evonik Patent Association

(57) **Zusammenfassung**

Gegenstand der Erfindung ist ein Verfahren zur Bereitstellung eines Kohlenmonoxid-haltigen Stroms. Das erfindungsgemäße Verfahren ist gekennzeichnet durch eine Auftrennung von Synthesegas in einen Wasserstoff-reichen Gasstrom und einen Kohlenmonoxid-reichen Gasstrom, der zu 85 Vol.-% oder mehr aus Kohlenmonoxid besteht, wobei die Auftrennung in einer Anordnung aus drei Membrantrennstufen erfolgt. Vor der Durchführung der Membrantrennung wird das Synthesegases zur Abtrennung von im Synthesegas vorhandenen Nebenkomponenten vorbehandelt.

## Beschreibung

Die vorliegende Erfindung betrifft ein Verfahren zur Bereitstellung eines Kohlenmonoxid-haltigen Stroms. Das erfindungsgemäße Verfahren ist gekennzeichnet durch eine Auftrennung von Synthesegas in einen Wasserstoff-reichen Gasstrom und einen Kohlenmonoxid-reichen Gasstrom, der zu 85 Vol.-% oder mehr aus Kohlenmonoxid besteht, wobei die Auftrennung in einer Anordnung aus mindestens drei Membrantrennstufen erfolgt. Vor der Durchführung der Membrantrennung wird das Synthesegases zur Abtrennung von im Synthesegas vorhandenen Nebenkomponenten vorbehandelt.

Verfahren zur Herstellung von Kohlenmonoxid-haltigen Strömen sind im Stand der Technik bekannt. Hauptsächlich wird Kohlenmonoxid durch die unvollständige Verbrennung von Kohlenstoff-haltigen Stoffen, beispielsweise die Kohlevergasung, die Kohlendioxid-Reformierung oder die partielle Oxidation von Kohlenwasserstoffen. Das Problem dabei ist, dass neben Kohlenmonoxid auch andere Stoffe wie Wasserstoff entstehen. Ein bekanntes Verfahren zur Herstellung von Kohlenmonoxid-haltigen Strömen mit geringen Anteilen an Wasserstoff ist der Otto-Prozess, der jedoch in großtechnischen Anlagen kaum eingesetzt werden kann.

Insofern werden üblicherweise Gasmischungen erhalten, die je nach Herstellverfahren unterschiedliche Anteile an Kohlenmonoxid enthalten. Soll jedoch ein Kohlenmonoxid-haltiger Strom erhalten werden, der einen hohen Anteil an Kohlenmonoxid aufweist und daher vergleichsweise rein ist, ist eine weitere Auftrennung der erhaltenen Gasmischung erforderlich. Ein bekanntes Verfahren zur Auftrennung von Gasgemischen ist beispielsweise die kryogene Aufteilung in die einzelnen Stoffe. Ein entsprechendes Verfahren ist beispielsweise in der DE 699 09 143 T2, wo die kryogene Auftrennung von Synthesegas offenbart ist.

Die kryogene Aufteilung ist jedoch ein recht teures und vergleichsweise aufwendiges Verfahren, weil sehr kalte Methanströme für die Absorption von CO vorhanden und eingesetzt werden müssen und/oder eine Auftrennung bei sehr kalten Temperaturen, z. B. mittels kryogener Rektifikation, erforderlich ist. Es besteht also ein Bedarf eines einfachen und vergleichsweise günstigen Verfahren, um Gasgemische wie Synthesegas aufzutrennen.

Die Aufgabe der vorliegenden Erfindung war demnach die Bereitstellung eines Verfahrens, mit dem auf einfache und sehr kostengünstige Weise ein Kohlenmonoxid-haltiger Strom erzeugt werden kann. Der Kohlenmonoxid-haltige Strom sollte dabei möglichst rein sein und das Verfahren sollte sich möglichst durch hohe Ausbeuten an Kohlenmonoxid, also nur geringen Verlusten an Kohlenmonoxid während des Trennverfahrens, auszeichnen.

Die Aufgabe konnte durch das erfindungsgemäße Verfahren nach Anspruch 1 gelöst werden. Bevorzugte Ausgestaltungen sind in den Unteransprüchen angegeben. Das erfindungsgemäße Verfahren ist ein Verfahren zur Auftrennung von Synthesegas in einen Wasserstoff-reichen Gasstrom und einen Kohlenmonoxid-reichen Gasstrom in einer Membrantrenneinheit, welche mindestens drei Membrantrennstufen umfasst, wobei im Synthesegas ein Verhältnis von Wasserstoff zu Kohlenmonoxid im Bereich von 70 : 30 bis 30 : 70, bezogen auf den jeweiligen Volumenanteil von Wasserstoff und Kohlenmonoxid im Synthesegas, vorliegt, und wobei das Verfahren die folgenden Schritte aufweist:
a. Bereitstellen des Synthesegases und Vorbehandeln des Synthesegases zur zumindest teilweisen Abtrennung von einer oder mehreren im Synthesegas vorhandenen Nebenkomponente(n);
b. Zuführen eines Feedstroms, der das vorbehandelte Synthesegas und einen Rezyklatstrom umfasst, zur ersten Trennstufe unter Erhalt eines ersten Retentats und eines ersten Permeats, wobei Kohlenmonoxid im ersten Retentat und Wasserstoff im ersten Permeat angereichert wird;
c. Zuführen des ersten Retentats zur zweiten Trennstufe unter Erhalt eines zweiten Retentats und eines zweiten Permeats, wobei das zweite Rententat als Kohlenmonoxid-reicher Gasstrom entnommen wird und zu 85 Vol.-% oder mehr aus Kohlenmonoxid besteht und wobei das zweite Permeat vor die erste Trennstufe zurückgeführt wird;
d. Zuführen des ersten Permeats zur dritten Trennstufe unter Erhalt eines dritten Retentats und eines dritten Permeats, wobei das dritte Permeat als Wasserstoff-reicher Gasstrom entnommen wird und zu 70 Vol.-% oder mehr aus Wasserstoff besteht und wobei das dritte Retentat vor die erste Trennstufe zurückgeführt wird,
wobei das zweite Permeat und das dritte Rententat zu einem einzigen Rezyklatstrom vereint werden und der Druck des Rezyklatstroms mithilfe eines Kompressor erhöht wird, bevor der Rezyklatstrom vor der ersten Trennstufe mit dem vorbehandelten Synthesegas zu dem in Schritt b) eingesetzten Feedstrom kombiniert wird, wobei das Verhältnis von vorbehandeltem Synthesegas zu Rezyklatstrom im Feedstrom im Bereich 4 : 1 bis 1 : 1,5, bevorzugt 3 : 1 bis 1 : 1,2, besonders bevorzugt 2,5 : 1 bis 1 : 1 bezogen auf den jeweiligen Normvolumenstrom von vorbehandeltem Synthesegas und Rezyklatstrom ist.

Das erfindungsgemäße Verfahren zeichnet sich folglich durch eine mindestens dreistufige Membrantrennung aus. Entsprechende Verfahren sind in vielen Fällen einfach auch in bestehende Anlagen zu integrieren, da entsprechende Vorrichtungen relativ platzsparend und einfach vom Aufbau sind. Weiterhin ist der Betrieb kostengünstig, weil energetisch anspruchsvolle Verfahrensschritte, z. B. die Verwendung von sehr kalten Stoffen oder Stoffgemischen, vermieden werden können.

Der Einsatzstoff für das erfindungsgemäße Verfahren sind typische Synthesegase, also Gasmischungen, die hauptsächlich Kohlenmonoxid und Wasserstoff enthalten. Entsprechende Synthesegase sind üblicherweise an chemischen Produktionsstandorten verfügbar. Das in der vorliegenden Erfindung bereitgestellte Synthesegas wird vorzugsweise bereits mit einem Druck im Bereich von 20 bis 80 bar bereitgestellt. Ein Verfahren mit dem Synthesegas mit einem solchen Druck bereitgestellt werden kann ist die Ölvergasung. Erfindungsgemäß bevorzugt ist, dass das Synthesegas vor der Membrantrennung nicht weiter komprimiert wird, also kein Kompressor für den eingesetzten Frischfeed aus Synthesegas vorliegt. Erfindungsgemäß liegt nur ein Kompressor im Rezyklatstrom vor.

Im Synthesegas liegt dabei ein Verhältnis von Wasserstoff zu Kohlenmonoxid im Bereich von 70 : 30 bis 30 : 70, vorzugsweise 60 : 40 bis 40 : 60, jeweils bezogen auf den jeweiligen Volumenanteil von Wasserstoff und Kohlenmonoxid im Synthesegas, vor. Es können aber Synthesegase eingesetzt werden, bei denen ein Verhältnis von Wasserstoff zu Kohlenmonoxid im Bereich von 55 : 45 bis 45 : 55, bezogen auf den jeweiligen Volumenanteil von Wasserstoff und Kohlenmonoxid im Synthesegas, vorliegt. Bevorzugte Synthesegase enthalten mindestens 30 Vol.-% an Kohlenmonoxid, vorzugsweise mindestens 40 Vol.-% an Kohlenmonoxid. Dadurch lassen sich auch mit vergleichsweise geringer Membranfläche hohe Reinheiten beim zweiten Retentat, also dem Kohlenmonoxid-reichen Gasstrom, erreichen.

Synthesegase enthalten weiterhin gewisse Mengen an Nebenkomponenten. Nebenkomponenten bei Synthesegasen können beispielsweise Wasser, Kohlendioxid, Schwefelverbindungen wie H₂S und COS, C2- oder höhere Kohlenwasserstoffe oder ähnliches sein. Das Synthesegas sollte vorzugsweise maximal 25 Vol.-%, vorzugsweise 20 Vol.-%, besonders bevorzugt weniger als 15 Vol.-% an Nebenkomponenten enthalten, wobei weniger Nebenkomponenten tendenziell zu einer besseren Trennbarkeit und höheren Reinheit des Kohlenmonoxid-haltigen Stroms führen. Es ist weiterhin bevorzugt, wenn im eingesetzten Synthesegas ein Verhältnis CO/CH₄ von mehr als 10 mol/mol, vorzugsweise mehr als 20 mol/mol und besonders bevorzugt mindestens 40 mol/mol vorliegt. Bestimmte Nebenkomponenten sollten vor der Membrantrennung aus dem Synthesegas zumindest teilweise entfernt werden. Dazu ist erfindungsgemäß die Vorbehandlung in Schritt a. vorgesehen, bei der eine oder mehrere Nebenkomponenten zumindest teilweise abgetrennt werden. Die Vorbehandlung kann aus einem oder mehreren separaten Schritten bestehen. Ein bekannter Prozess zur Entfernung von Verunreinigungen wie CO₂ und H₂S aus dem Rohsynthesegas ist eine Gegenstromabsorption mit einem regenerativen Lösungsmittel, z. B. einem chemischen Lösungsmittel auf Alkanolaminbasis (z. B. DIPA) oder Hybridlösungsmittel (chemische und physikalische Lösungsmittel) wie Mischungen aus Alkanolamin (z. B. DIPA) und Sulfolan.

Es ist möglich mit dem erfindungsgemäßen Verfahren sehr reine Kohlenmonoxid-haltige Ströme, die im Rahmen der vorliegenden Erfindung auch als Kohlenmonoxid-reiche Gasströme bezeichnet werden, zu erhalten. Diese Ströme fallen innerhalb des in dieser Erfindung beschriebenen Verfahrens als zweites Retentat in der zweiten Trennstufe an und bestehen zu mindestens 85 Vol.-% oder mehr aus Kohlenmonoxid. Es ist jedoch bevorzugt, wenn das zweite Rententat, also der Kohlenmonoxid-reiche Gasstrom, zu mindestens 87 Vol.-% oder mehr aus Kohlenmonoxid, vorzugsweise zu mindestens 91 Vol.-% aus Kohlenmonoxid und besonders bevorzugt zu mindestens 95 Vol.-% oder mehr aus Kohlenmonoxid besteht. Es ist weiterhin besonders bevorzugt, wenn das Verhältnis H₂/CO im zweiten Rententat, also im Kohlenmonoxid-reichen Gasstrom, kleiner als 0,05 mol/mol ist.

Auch wenn das Ziel der vorliegenden Erfindung ist, als zweites Retentat einen Kohlenmonoxid-haltigen Strom mit hoher Reinheit zu erhalten, ist es technisch kaum möglich einen reinen Kohlenmonoxid-Strom zu erzeugen. Je nach Einsatzzweck für den als zweites Retentat erhaltenen Kohlenmonoxid-haltigen Strom ist eine besonders hohe Reinheit auch gar nicht erforderlich. Es ist jedoch zumindest bevorzugt, dass nur wenig Wasserstoff im Kohlenmonoxid-haltigen Strom vorhanden ist. Der als zweites Retentat anfallende Kohlenmonoxid-haltige Strom enthält vorzugsweise höchstens 2 Vol.-% an Wasserstoff, besonders bevorzugt höchstens 1 Vol.-% an Wasserstoff.

Es ist bereits erwähnt worden, dass das Kernziel der vorliegenden Erfindung die Bereitstellung eines Kohlenmonoxid-haltigen Stroms als zweites Retentat, wobei der Strom eine hohe Reinheit aufweist, d. h. zu mindestens 85 Vol.-% oder mehr aus Kohlenmonoxid besteht. Bei dem erfindungsgemäßen Verfahren fällt jedoch als drittes Permeat ein Wasserstoff-reicher Gasstrom an, der zu 70 Vol.-% oder mehr aus Wasserstoff besteht. Die Reinheit des als das dritte Permeat anfallenden Wasserstoff-reichen Gasstroms ist im Rahmen der vorliegenden Erfindung ist insbesondere weniger hoch priorisiert als die Reinheit des zweiten Retentat. Es ist sogar bevorzugt, zu versuchen alle im Synthesegas vorhandenen anderen Gase in den Wasserstoff-reicher Gasstrom zu bekommen, insbesondere und zumindest das Kohlendioxid. Demgemäß besteht das dritte Permeat in Abhängigkeit von der Zusammensetzung des Synthesegases neben dem Wasserstoff aus weiteren gasförmigen Stoffen wie Kohlendioxid.

Der als das dritte Permeat anfallende Wasserstoff-reiche Gasstrom kann weiterhin geringe Mengen an Kohlenmonoxid enthalten. Das kann auch deshalb erforderlich sein, um die notwendige Trennwirkung zu verstärken und ein möglichst reines zweites Retentat zu erreichen. Es ist jedoch so, dass höhere Anteil an Kohlenmonoxid im dritten Permeat mit einer schlechteren Ausbeute einhergehen. Es ist somit anhand der jeweiligen Zusammensetzung des Synthesegases abzuwägen, wie das Verfahren betrieben wird. Es ist in dieser Hinsicht allgemein bevorzugt, dass das dritte Permeat weniger als 3 Vol.- % an Kohlenmonoxid, vorzugsweise weniger als 2 Vol.-% an Kohlenmonoxid, besonders bevorzugt weniger als 1 Vol.-% an Kohlenmonoxid enthält.

Die Membrantrennung nach dem erfindungsgemäßen Verfahren ist ein mindestens dreistufiges Verfahren, bei dem die Stufen speziell miteinander verschaltet sind. Die erste Trennstufe ist eine Membrantrennstufe zur Auftrennung des Feedstroms unter Erhalt eines ersten Retentats und eines ersten Permeats, wobei Kohlenmonoxid im ersten Retentat und Wasserstoff im ersten Permeat angereichert wird. Die zweite Trennstufe ist eine Membrantrennstufe, die im Vergleich mit der ersten Trennstufe identisch oder unterschiedlich aufgebaut sein kann, zur Auftrennung des ersten Retentats unter Erhalt eines zweiten Retentats und eines zweiten Permeats. Die dritte Trennstufe ist eine Membrantrennstufe, die im Vergleich mit der ersten und der zweiten Trennstufe identisch oder unterschiedlich aufgebaut sein kann, zur Auftrennung des ersten Permeats unter Erhalt eines dritten Retentats und eines dritten Permeats. Es ist aber auch denkbar, dass die Membrantrennung in vier Stufen oder in mehr als vier Stufen erfolgt.

Als Membranen können in den einzelnen Membrantrennstufen geeignete Gasseparationsmembran eingesetzt werden, an denen das Synthesegas aufgrund der für die einzelnen Gase in der Mischung unterschiedlichen Permeanzen (= Stoffstrom pro Zeiteinheit, Fläche, Differenzdruck und Schichtdicke) aufgetrennt werden können. Das in den Gasseparationsmembranen verwendete Membranmaterial kann beispielsweise in Form von Hohlfasern oder Flachmembranen eingesetzt werden. Vorzugsweise werden mehrere derartiger Gasseparationsmembranen in einem Trennmodul zusammengefasst. Alle drei Membrantrennstufen können aus einem oder mehreren Trennmodulen bestehen, wobei bei Vorliegen von mehreren Trennmodulen, diese Trennmodule innerhalb einer Trennstufe parallel und/oder seriell verschaltet sind. Der Aufbau und die Anzahl der Trennmodule kann dabei zwischen den verschiedenen Membrantrennstufen variieren.

Die erfindungsgemäße Vorrichtung bzw. das erfindungsgemäße Verfahren grundsätzlich mit allen geeigneten Membranmaterialien realisiert werden. Geeignete Membranmaterialien für das vorliegende Verfahren sind solche, mit denen es möglich ist Synthesegase in einen Wasserstoff-reichen Gasstrom und einen Kohlenmonoxid-reichen Gasstrom aufzutrennen. Membranmaterialien für die Trennung von Mischungen von mehreren Stoffen können aus mehreren Schichten bestehen, bspw. bei Kompositmembranen, oder aus einem einzelnen Material bestehen, bspw. bei integral-asymmetrischen Membranen. Die für die eigentliche Abtrennung maßgebliche Schicht wird auch als trennaktive Schicht bezeichnet, durch die der Wasserstoff schneller permeiert als das Kohlenmonoxid, wodurch es zu der gewünschten Trennung kommt. Als Membranmaterialien kann insbesondere für die trennaktive Schicht ein Material, ausgewählt aus der Gruppe, bestehend aus Polyimiden, Polyamiden, Polysulfonen, Celluloseacetaten und deren Derivaten, Polyphenylenoxiden, Polysiloxanen, Polymeren mit intrinsischer Mikroporosität, Mixed Matrix Membranen, Facilitated Transport Membranen, Polyethylenoxiden, Polypropylenoxiden, Kohlenstoffmembranen, Zeolithe und Mischungen daraus, eingesetzt werden.

In einer bevorzugten Ausführungsform der vorliegenden Erfindung werden für die trennaktive Schicht oder für das komplette Membranmaterial ein Polyimid eingesetzt. Geeignete Polyimid-Membranen sind beispielsweise Polyimid P84 und Polyimid P84 HT von der Evonik Fibres GmbH oder Mischungen von Polyimid P84 und/oder Polyimid P84 HT mit anderen Membranmaterialien. Ein Verfahren zur Herstellung dieser Membranen wird ist in der WO 2011/009919 A1 offenbart, wobei alle der dort offenbarten Membranmaterialien im hier beschriebenen Verfahren eingesetzt werden können.

Die Membranmaterialien werden im Rahmen der vorliegenden Erfindung bevorzugt in Form von Hohlfasermembranen und/oder Flachmembranen verwendet. Weiterhin bevorzugt werden mehrere der Hohlfasermembranen und/oder Flachmembranen zu Trennmodulen verbaut, die in den drei Trennstufen zum Einsatz kommen. Als Module können alle in der Technik bekannten Gasseparationsmodule wie zum Beispiel Hohlfasermodule, Kapillarmodule, Rohrmodule, Spiralwickelmodule, Kissenmodule, Plattenmodule oder Taschenmodule verwendet werden.

Die Trennmodule weisen vorzugsweise eine Reinstoffselektivität bei 25 °C für Wasserstoff/Kohlenmonoxid von mindestens 25, weiterhin bevorzugt von mindestens 30, besonders bevorzugt von mindestens 40 auf. Höher selektive Membranen haben den Vorteil, dass die Trennung effektiver wird und weniger zweites Permeat aus zweiten Trennstufe und/oder weniger drittes Retentat aus der dritten Trennstufe rückgeführt werden muss. Es sollte daher klar sein, dass der erfindungsgemäße Prozess mit selektiveren Membranen teils deutlich wirtschaftlicher geführt werden kann. Trotzdem können auch weniger selektive Membranen eingesetzt werden, solange der eigentliche Zweck, also die Bereitstellung eines möglichst reinen Kohlenmonoxid-Stroms, erreicht werden kann.

Die Trennstufen der vorliegenden Erfindung können weiterhin über ihre Kapazität definiert werden. Als Kapazität ist im Rahmen der vorliegenden Erfindung die Separationskapazität zu verstehen ist, die sich anhand der Membranfläche pro Trennmodul und der Anzahl der Trennmodule pro Trennstufe multipliziert mit der Wasserstoffpermeanz der eingesetzten Membran errechnen lässt. Es ist erfindungsgemäß bevorzugt, dass die zweite Trennstufe oder die dritte Trennstufe die höchste Kapazität aufweist. Besonders bevorzugt weist die zweite Trennstufe die höchste Kapazität auf.

Als Triebkraft für die Auftrennung des Synthesegases in den Trennmodulen wird eine Druckdifferenz zwischen der Retentatseite und der Permeatseite, der auch als Transmembrandruck bezeichnet wird, in den jeweiligen drei Trennstufen erzeugt. Die Druckdifferenz basiert insbesondere auf dem Druck des Feedstroms, der zu ersten Trennstufe geführt wird. Der Feedstrom kann dazu mittels eines geeigneten Kompressors komprimiert werden. Gemäß einer bevorzugten Ausführungsform der vorliegenden Erfindung werden das vorbehandelte Synthesegas und der Feedstrom jedoch nicht komprimiert, sondern bereits mit dem richtigen Druck bereitgestellt. Auf Seiten des Feeds liegt deshalb vorzugsweise kein Kompressor vor. Das vorliegend beschriebene Verfahren weist jedoch einen Kompressor für den Rezyklatstrom auf, um den Druck des Rezyklatstroms auf den Druck des vorbehandelten Synthesegases zu bringen, bevor diese zum Feedstrom der ersten Membrantrennstufe vereint werden. Zur Erzeugung der Druckdifferenz kann außerdem auch eine Vakuumpumpe auf der Permeatseite einer Trennstufe vorhanden sein, ggf. auch zusätzlich zu einem Kompressor.

Für die erste Trennstufe liegen vorzugsweise die folgenden Druckverhältnisse vor. Der retentatseitige Druck der ersten Trennstufe, also der Druck, mit dem der Feedstrom zur ersten Trennstufe geführt wird, beträgt vorzugsweise zwischen 20 und 80 bar, weiterhin bevorzugt zwischen 25 und 65 bar, besonders bevorzugt zwischen 30 und 45 bar. Der permeatseitige Druck der ersten Trennstufe beträgt vorzugsweise zwischen 2,5 und 30 bar, weiterhin bevorzugt zwischen 3,0 und 26 bar, besonders bevorzugt zwischen 3,3 und 21 bar. Dieser permeatseitige Druck kann beispielsweise über ein geeignetes Druckminderventil eingestellt werden. Daraus ergibt sich, dass der Transmembrandruck in der ersten Trennstufe vorzugsweise zwischen 9 bis 75 bar, weiterhin bevorzugt zwischen 9 bis 60 bar, besonders bevorzugt zwischen 13 bis 40 bar beträgt. Es versteht, dass sich der Transmembrandruck als Druckdifferenz zwischen Retentatseite und Permeatseite bestimmte Werte nur dann annehmen kann, wenn ein entsprechend hoher retentatseitige Druck oder entsprechend niedriger permeatseitige Druck vorliegt. Die Auswahl geeigneter Retentat- und Permeatdrücke zur Erreichung eines technisch sinnvollen Transmembrandrucks ist dem Fachmann geläufig. Die Angabe bar meint im hier immer bar absolut (Abk.: bara).

Für die zweite Trennstufe liegen vorzugsweise die folgenden Druckverhältnisse vor. Der retentatseitige Druck der zweiten Trennstufe, also der Druck, der mit möglichen kleineren Druckverlusten der Retentatseite der ersten Trennstufe entspricht, beträgt vorzugsweise zwischen 19,5 und 79,5 bar, weiterhin bevorzugt zwischen 24,5 und 64,5 bar, besonders bevorzugt zwischen 29,5 und 45 bar. Der permeatseitige Druck der zweiten Trennstufe beträgt vorzugsweise zwischen 2,0 und 30 bar, weiterhin bevorzugt zwischen 2,5 und 26 bar, besonders bevorzugt zwischen 2,8 und 21 bar. Dieser permeatseitige Druck kann beispielsweise über ein geeignetes Druckminderventil eingestellt werden. Daraus ergibt sich, dass der Transmembrandruck in der zweiten Trennstufe vorzugsweise zwischen 9 und 75 bar, weiterhin bevorzugt zwischen 9 und 60 bar, besonders bevorzugt zwischen 13 und 40 bar beträgt. Es versteht, dass sich der Transmembrandruck als Druckdifferenz zwischen Retentatseite und Permeatseite bestimmte Werte nur dann annehmen kann, wenn ein entsprechend hoher retentatseitige Druck oder entsprechend niedriger permeatseitige Druck vorliegt. Die Auswahl geeigneter Retentat- und Permeatdrücke zur Erreichung eines technisch sinnvollen Transmembrandrucks ist dem Fachmann geläufig. Die Angabe bar meint im hier immer bar absolut (Abk.: bara).

Für die dritte Trennstufe liegen vorzugsweise die folgenden Druckverhältnisse vor. Der retentatseitige Druck der dritten Trennstufe, also der Druck, der mit möglichen kleineren Druckverlusten der Permeatseite der ersten Trennstufe entspricht, beträgt vorzugsweise zwischen 1,5 und 30 bar, weiterhin bevorzugt zwischen 2,0 und 26 bar, besonders bevorzugt zwischen 2,3 und 21 bar beträgt. Der permeatseitige Druck der dritten Trennstufe beträgt vorzugsweise zwischen 200 mbar und 10 bar, weiterhin bevorzugt zwischen 300 mbar und 8 bar, besonders bevorzugt zwischen 500 mbar und 5 bar. Daraus ergibt sich, dass der Transmembrandruck in der dritten Trennstufe vorzugsweise zwischen 1,0 und 29,8 bar, weiterhin bevorzugt zwischen 1,0 und 25,8 bar, besonders bevorzugt zwischen 1,0 und 20,5 bar beträgt. Es versteht, dass sich der Transmembrandruck als Druckdifferenz zwischen Retentatseite und Permeatseite bestimmte Werte nur dann annehmen kann, wenn ein entsprechend hoher retentatseitige Druck oder entsprechend niedriger permeatseitige Druck vorliegt. Die Auswahl geeigneter Retentat- und Permeatdrücke zur Erreichung eines technisch sinnvollen Transmembrandrucks ist dem Fachmann geläufig. Die Angabe bar meint im hier immer bar absolut (Abk.: bara).

Das zweite Permeat aus der zweiten Trennstufe und das dritte Rententat aus der dritten Trennstufe werden erfindungsgemäß zu einem einzigen Rezyklatstrom vereint und mit dem vorbehandelten Synthesegas zum Feedstrom kombiniert. Der Druck des Rezyklatstroms wird vor dem Kombinieren mit dem vorbehandelten Synthesegas mithilfe eines Kompressor erhöht, um den Rezyklatstrom auf das gleiche Druckniveau zu bringen. Das Verhältnis von vorbehandeltem Synthesegas zu Rezyklatstrom im Feedstrom liegt dabei im Bereich von 4 : 1 bis 1 : 1,5, bevorzugt von 3 : 1 bis 1 : 1,2, besonders bevorzugt von 2,5 : 1 bis 1 : 1, jeweils bezogen auf den jeweiligen Normvolumenstrom von vorbehandeltem Synthesegas und Rezyklatstrom. Es sollte berücksichtigt werden, dass das maximal oder minimal mögliche Verhältnis von vorbehandeltem Synthesegas zu Rezyklatstrom im Feedstrom in gewisser Weise von der Reinstoffselektivität der eingesetzten Membranmodule abhängt. Je höher die Reinstoffselektivität ist, desto geringer kann die Menge an Rezyklat sein. Entsprechende Abhängigkeiten sind dem Fachmann jedoch bekannt oder lassen sich recht einfach ermitteln.

Das erfindungsgemäße Verfahren kann grundsätzlich bei jeder geeigneten Temperatur stattfinden, um also den erfindungsgemäßen Zweck der Bereitstellung eines möglichst reinen Kohlenmonoxid-Stroms zu erfüllen. Es ist jedoch bevorzugt, dass bei dem erfindungsgemäßen Verfahren in allen drei Trennstufen eine Temperatur im Bereich von 15 bis 100 °C, besonders bevorzugt zwischen 25 und 60 °C vorliegt. Die Temperaturen in den einzelnen Trennstufen kann identisch sein, wird sich aber in den meisten Fällen ohne entsprechende Wärmezufuhr aufgrund der Gasentspannung über die Membran unterscheiden. In Abhängigkeit von der Temperatur des vorbehandelten Synthesegases bzw. des Feedstroms und der gewünschten Temperatur in der ersten Trennstufe kann der Feedstrom vor dem Zuführen in die erste Trennstufe erwärmt oder gekühlt werden. In einer bevorzugten Ausführungsform wird der Feedstrom vor dem Zuführen in die erste Trennstufe erwärmt. Die Erwärmung des Feedstroms kann beispielsweise in einem Wärmetauscher erfolgen. Als Wärmeträgermedium können beispielsweise Prozessdampf oder ein geeignetes Wärmeträgeröl verwendet werden. Es ist aber auch möglich, dass die Erwärmung des Feedstroms elektrisch erfolgt.

Das bei dem Verfahren erhaltene zweite Retentat, also der anfallende Kohlenmonoxid-haltige Strom, kann erfindungsgemäß für die chemische Synthese eingesetzt werden. Syntheseverfahren, bei denen CO benötigt wird, sind dem Fachmann bekannt. In einer besonders bevorzugten Ausführungsform der vorliegenden Erfindung wird das zweite Retentat bei der Alkoxycarbonylierung eingesetzt. Bei der Alkoxycarbonylierung wird ein Kohlenwasserstoff, der mindestens eine Mehrfachbindung, vorzugsweise mindestens eine olefinische Doppelbindung aufweist, mit Kohlenmonoxid und einem Alkohol zu den entsprechenden Estern umgesetzt. Insofern ist ein weiterer Gegenstand der vorliegenden Erfindung ein integriertes Verfahren zur Alkoxycarbonylierung von C2- bis C20-Kohlenwasserstoffen mit mindestens einer olefinischen Doppelbindung mit einem Kohlenmonoxid-Strom und mit einem Alkohol in Gegenwart eines homogenen Katalysatorsystems in einer Reaktionszone, wobei das in Schritt b) des erfindungsgemäßen Verfahrens bereitgestellte zweite Retentat als Kohlenmonoxid-Strom eingesetzt wird.

Bei der Alkoxycarbonylierung des integrierten Verfahrens der vorliegenden Erfindung werden vorzugsweise C3- bis C16-Kohlenwasserstoffe, besonders bevorzugt C4- bis C12-Kohlenwasserstoffe mit mindestens einer Mehrfachbindung, vorzugsweise mindestens einer olefinischen Doppelbindung eingesetzt werden. Besonders bevorzugte Kohlenwasserstoffe, die bei der Alkoxycarbonylierung des integrierten Verfahrens eingesetzt werden, weisen nur eine olefinische Doppelbindung auf, insbesondere n-Alkene und iso-Alkene mit 2 bis 20 Kohlenstoffatomen, vorzugsweise 3 bis 16 Kohlenstoffatomen, besonders bevorzugt 4 bis 12 Kohlenstoffatomen. Die eingesetzten Kohlenwasserstoffe sind vorzugsweise unsubstituiert.

Der bei der Alkoxycarbonylierung des integrierten Verfahrens eingesetzte Alkohol ist ein Mono- oder Polyalkohol (zwei oder mehr OH-Gruppe) mit 1 bis 15 Kohlenstoffatomen, besonders bevorzugt 1 bis 10 Kohlenstoffatomen oder eine Mischung aus zwei oder mehr Mono- und/oder Polyalkoholen. In einer bevorzugten Ausführungsform ist der Polyalkohol ein Diol, Triol oder Tetrol, vorzugsweise ein Diol oder Triol mit der vorgenannten Anzahl an Kohlenstoffatomen. Geeignete Alkohole für die Umsetzung in Schritt a) sind Methanol, Ethanol, 1-Propanol, 1-Butanol, 1-Pentanol, 1-Hexanol, 2-Propanol, tert-Butanol, 3-Pentanol, 2-Propylheptanol, Cyclohexanol, Phenol oder Mischungen daraus, vorzugsweise Ethanol, 1-Propanol, 1-Butanol, 1-Pentanol, 1-Hexanol, 2-Propanol, tert-Butanol, 3-Pentanol, 2-Propylheptanol.

Der bei der Alkoxycarbonylierung des integrierten Verfahrens eingesetzte Alkohol wird, wenn es sich um einen Monoalkohol handelt, in einem Molverhältnis zum eingesetzten Kohlenwasserstoff (Monoalkohol : Kohlenwasserstoff) von 2 bis 20, vorzugsweise von 3 bis 10 und besonders bevorzugt von 4 bis 6 eingesetzt. Der Monoalkohol wird somit - bezogen auf den eingesetzten Kohlenwasserstoff - im molaren Überschuss hinzugefügt. Dadurch kann der Alkohol sowohl als Edukt für die Carbonylierung als auch als Lösemittel fungieren. Der bei der Alkoxycarbonylierung des integrierten Verfahrens eingesetzte Alkohol wird, wenn es sich um einen Polyalkohol handelt, in einem Molverhältnis zum eingesetzten Kohlenwasserstoff (Kohlenwasserstoff: Polyalkohol) von 2 bis 20, vorzugsweise von 3 bis 10 und besonders bevorzugt von 4 bis 8 eingesetzt. Der Polyalkohol wird bezogen auf den eingesetzten Kohlenwasserstoff also im molaren Unterschuss hinzugefügt.

Die Alkoxycarbonylierung des integrierten Verfahrens wird in Gegenwart eines homogenen Katalysatorsystems durchgeführt. Bevorzugt umfasst das homogene Katalysatorsystem zumindest ein Metall aus der Gruppe 8 bis 10 des Periodensystems der Elemente (PSE) oder eine Verbindung davon, einen phosphorhaltigen Liganden und eine Säure als Co-Katalysator.

Das Metall aus der Gruppe 8 bis 10 des PSE ist vorzugsweise Palladium. Das Palladium wird bevorzugt in Form einer Vorläuferverbindung als Palladiumverbindung eingesetzt, die durch den phosphorhaltigen Liganden koordiniert wird. Beispiele für Palladiumverbindungen, die als Vorläuferverbindungen eingesetzt werden können, sind Palladiumchlorid [PdCl₂], Palladium(II)-Acetylacetonat [Pd(acac)₂], Palladium(II)-Acetat [Pd(OAc)₂], Dichloro-(1,5-cyclooctadien)palladium(II) [Pd(cod)Cl₂], Bis(dibenzylideneaceton)palladium(0) [Pd(dba)₂], Tris(dibenzylideneaceton)dipalladium(0) [Pd₂(dba)₃] Bis(acetonitril)-dichloropalladium(II) [Pd(CH₃CN)₂Cl₂], Palladium(cinnamyl)-dichlorid [Pd(cinnamyl)Cl₂]. Vorzugsweise kommen die Verbindungen [Pd(acac)₂] oder [Pd(OAc)₂] zum Einsatz. Die Metallkonzentration von Palladium bei der Alkoxycarbonylierung beträgt vorzugsweise zwischen 0,01 und 0,6 Mol-%, bevorzugt zwischen 0,03 und 0,3 Mol-%, besonders bevorzugt zwischen 0,04 und 0,2 Mol-% bezogen auf die Stoffmenge des eingesetzten Kohlenwasserstoffs.

Geeignete phosphorhaltige Liganden des erfindungsgemäßen Katalysatorsystems weisen vorzugsweise eine Bidentat-Struktur auf. Bevorzugte phosphorhaltige Liganden für die das erfindungsgemäße Katalysatorsystem sind benzolbasierte Diphosphinverbindungen, wie sie beispielsweise in der EP 3 121 184 A2 offenbart worden sind. Die Liganden können in einer Vorreaktion mit dem Palladium kombiniert werden, sodass der Palladium-Ligand-Komplex zur Reaktionszone geführt wird, oder in situ zur Reaktion gegeben und dort mit dem Palladium kombiniert werden. Das Molverhältnis von Ligand : Metall bei der Alkoxycarbonylierung 1 : 1 bis 10 : 1, vorzugsweise 2 : 1 bis 6 : 1, besonders bevorzugt 3 : 1 bis 5 : 1 betragen.

Das homogene Katalysatorsystem umfasst weiterhin eine Säure, wobei es sich insbesondere um eine Brönsted- oder eine Lewis-Säure handelt. Als Lewis-Säure können insbesondere Aluminiumtriflat, Aluminiumchlorid, Aluminiumhydrid, Trimethylaluminium, Tris(pentafluorophenyl)boran, Bortrifluorid, Bortrichlorid oder Mischungen daraus eingesetzt werden. Von den genannten Lewis-Säuren wird bevorzugt Aluminiumtriflat eingesetzt. Die Lewis-Säure wird vorzugsweise in einem Molverhältnis Lewis-Säure : Ligand von 1 : 1 bis 20 : 1, vorzugsweise 2 : 1 bis 15 : 1, besonders bevorzugt 5 : 1 bis 10 : 1 hinzugegeben.

Geeignete Brönsted-Säuren haben vorzugsweise eine Säurestärke von pKs ≤ 5, besonders bevorzugt eine Säurestärke von pKs ≤ 3. Die angegebene Säurestärke pKs bezieht sich auf den bei Normalbedingungen (25°C, 1,01325 bar) bestimmten pKs-Wert. Bei einer mehrprotonigen Säure bezieht sich die Säurestärke pKs im Rahmen dieser Erfindung auf den pKs-Wert des ersten Protolyseschrittes. Die Brönsted-Säure wird vorzugsweise in einem Molverhältnis Brönsted-Säure : Ligand von 1 : 1 bis 15 : 1, vorzugsweise 2 : 1 bis 10 : 1, besonders bevorzugt 3 : 1 bis 5 : 1 hinzugegeben.

Als Brönsted-Säuren können insbesondere Perchlorsäure, Schwefelsäure, Phosphorsäure, Methylphosphonsäure oder Sulfonsäuren eingesetzt werden. Geeignete Sulfonsäuren sind beispielsweise Methansulfonsäure, Trifluormethansulfonsäure, tert-Butansulfonsäure, p-Toluolsulfonsäure (PTSA), 2-Hydroxypropan-2-sulfonsäure, 2,4,6-Trimethylbenzolsulfonsäure und Dodecylsulfonsäure. Besonders bevorzugte Säuren sind Schwefelsäure, Methansulfonsäure, Trifluormethansulfonsäure und p-Toluolsulfonsäure. Vorzugsweise handelt es sich bei der Säure um Schwefelsäure. Carbonsäure sind hingegen weniger bis gar nicht geeignet.

Die Alkoxycarbonylierung des integrierten Verfahrens wird vorzugsweise bei einer Temperatur von 25 bis 140 °C, weiterhin bevorzugt bei einer Temperatur von 80 bis 120 °C und besonders bevorzugt bei einer Temperatur von 90 bis 110 °C durchgeführt. Der Druck bei der Alkoxycarbonylierung kann zwischen 5 und 60 bar, vorzugsweise zwischen 10 und 40 bar, besonders bevorzugt zwischen 15 und 30 bar betragen.

Die Alkoxycarbonylierung des integrierten Verfahrens findet in einer geeigneten Reaktionszone statt. Die Reaktionszone für die Umsetzung umfasst mindestens einen Reaktor, kann aber auch aus zwei oder mehr Reaktoren bestehen. Der mindestens eine Reaktor kann insbesondere aus der Gruppe, bestehend aus einem Rührkesselreaktor, einem Schlaufenreaktor, einem Jet-Loop-Reaktor, einem Blasensäulenreaktor oder Kombinationen daraus, ausgewählt sein. Sind mehrere Reaktoren vorhanden können die Reaktoren gleich oder unterschiedlich sein.

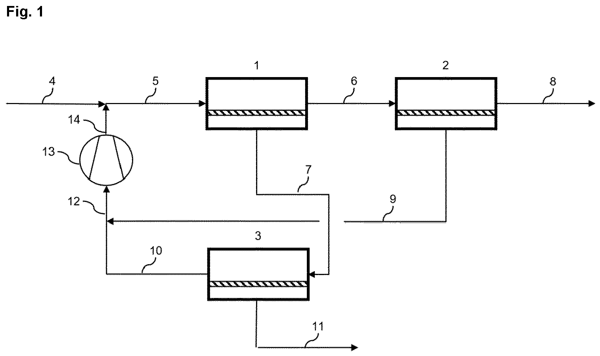

In Fig. 1 der vorliegenden Erfindung wird eine bevorzugte Ausführungsform der vorliegenden Erfindung gezeigt. Dort wird ein vorbehandeltes Synthesegas (4) mit einem Rezyklatstrom (14) kombiniert und als Feedstrom (5) zur ersten Membrantrennstufe (1) geführt. Das dabei erhaltene erste Retentat (6), in dem CO angereichert wird, wird zur zweiten Membrantrennstufe (2) geführt und einer weiteren Membrantrennung unterworfen. Das in der zweiten Membrantrennstufe (2) erhaltenen zweite Retentat (8) ist der Kohlenmonoxid-reiche Gasstrom, der entnommen wird und zu 85 Vol.-% oder mehr aus Kohlenmonoxid besteht. Das in der ersten Membrantrennstufe (1) erhaltene erste Permeat (7) wird zur dritten Membrantrennstufe (3) geführt. Das in der dritten Membrantrennstufe (3) erhaltene dritte Permeat (11) ist der Wasserstoff-reiche Gasstrom, der entnommen wird und zu 70 Vol.-% oder mehr aus Wasserstoff besteht. Das aus der zweiten Membrantrennstufe (2) erhaltene zweite Permeat (9) und das aus der dritten Membrantrennstufe (3) erhaltene dritte Retentat werden vereinigt und als gemeinsames Rezyklat (12) mittels eines Kompressors (13) komprimiert, bevor sie mit dem vorbehandelten Synthesegas (4) kombiniert werden.

Nachfolgend soll die vorliegende Erfindung anhand von Beispielen erläutert werden. Diese Beispiele stellen konkrete Ausführungsformen dar, die der Erläuterung dienen, nicht jedoch einschränkend zu verstehen sind.

### Beispiele

Die nachfolgenden Beispiele, bei denen jeweils Synthesegase aufgetrennt worden sind, wurden computerunterstützt simuliert. Dabei wurde simuliert, dass die jeweils eingesetzten Synthesegase durch eine Membrantrenneinheit mit drei Membrantrennstufen gemäß Fig. 1 aufgetrennt wurden. Die Untersuchungen sind in der kommerziellen Simulationsumgebung Aspen Custom Modeler durchgeführt worden. Das dort implementierte Membranmodell beruht auf dem bekannten Lösungs-Diffusionsansatz, der in der Trennung von Gasen mittels dichter polymerer Membranen eingesetzt wird. Als Triebkraft wird die Partialdruckdifferenz der Komponenten zwischen Feed und Permeat angenommen. Das Membranmodell ermöglicht eine geeignete Diskretisierung eines Membranmoduls über die Lauflänge und nimmt einen Gegenstrom an. Die Temperaturen in den drei Membrantrennstufen bei der Simulation wurde jeweils auf 40 °C eingestellt. Die Permeanzen für die einzelnen Membrankomponenten sind den jeweiligen Beispielen zu entnehmen.

Es werden konstante Permeanzen angenommen. Der Einfluss der Temperatur auf die Permeanzen und Selektivitäten wurde vernachlässigt. Auch nicht ideale Effekte wie. z.B. Druckverlust im Modul, Temperatur- und Konzentrationspolarisation, sowie der Joule-Thomson-Effekt blieben ebenfalls unberücksichtigt, weil diese Effekte keinen signifikanten Einfluss auf die allgemeinen Erkenntnisse haben. Weiterhin wurde die Vorbehandlung bei der Simulation nicht berücksichtigt, weil sich die Zusammensetzung der Ströme bei der Simulation so einstellen ließ, wie sie sich nach einer Vorbehandlung einstellen würde.

### Beispiel 1: Auftrennung eines Synthesegases (H₂ : CO = ca. 50 : 50) an einer Membran mit der H2/CO Selektivität von 40

Die Simulation erfolgte wie oben beschrieben. Das Verhältnis von H₂ zu CO im als Feed eingesetzten Synthesegas lag bei etwa 50 : 50 (vgl. Zusammensetzung des Feeds in Tabelle 2). Das Synthesegas wird bei einem Druck von 40 bara (bara = bar absolut) bereitgestellt. Als Membran wurde eine Membran mit den folgenden üblichen Permeanzen und Selektivitäten eingesetzt (vgl. WO 2020/079403 A1).

**Tabelle 1: Übersicht über übliche Permeanzen und Selektivitäten für bestimmte Gase**

| Komponente | H₂ | CO | N₂ | CH₄ | CO₂ |
|---|---|---|---|---|---|
| Permeanz [GPU*] | 200 | 5 | 4 | 2 | 50 |
| Selektivität [H₂/Gas] | 1 | 40 | 50 | 100 | 4 |

wobei 1 GPU = 1 × 10⁻⁶ cm³(STP)/ (cm²•s•cmHg).

Die erste Membrantrennstufe ist mit einer Fläche von 1063 m² realisiert. Der Permeatdruck der ersten Trennstufe beträgt 6,3 bara, so dass sich ein transmembraner Druck von 33,7 bar in der 1. Stufe ergibt. Das Retentat der ersten Trennstufe wird mit der zweiten Membrantrennstufe prozessiert. Die Membranfläche der zweiten Trennstufe ist mit 3815 m² größer als in der ersten Trennstufe. Das Permeat der zweiten Trennstufe weist einen Druck von 6,3 bara auf. Der transmembrane Druck ist demzufolge 33,7 bar. Das Permeat der ersten Trennstufe wird mit der dritten Membrantrennstufe prozessiert. Die dritte Membrantrennstufe hat eine Fläche von 2126 m². In diesem Beispiel hat die zweite Trennstufe somit die höchste Kapazität. Bei einem Permeatdruck von 1,25 bara in der dritten Trennstufe ergibt sich ein Transmembrandruck von 5 bar. Das Retentat der dritten Trennstufe wird mit dem Permeat der zweiten Trennstufe zum Rezyklat vereinigt und mittels Kompressors auf einen Druck von 40 bara gebracht. Anschließend wird das Rezyklat mit dem Synthesegas vereinigt auf die erste Membrantrennstufe geschickt. Der Normvolumenstrom des Synthesegases beträgt im vorliegenden Beispiel 10000 Nm³/h und der Normvolumenstrom des Rezyklats beträgt 5000 Nm³/h. Somit ergibt sich ein Verhältnis von Synthesegas zu Rezyklatstrom von 2 : 1. Insgesamt wird ein Normvolumenstrom des CO-Produkts (zweites Retentat) von 5059 Nm³/h erhalten. Die CO-Ausbeute der Membrantrennung beträgt 99,55 % und das CO-Produkt (zweites Retentat) weist eine Reinheit von 97,73% auf. Die Zusammensetzungen der einzelnen Ströme sind in Tabelle 2 gezeigt.

**Tabelle 2: Zusammensetzung der jeweiligen Ströme in Beispiel 1 / in Klammern sind die jeweiligen Bezugszeichen aus Fig. 1 angegeben.**

| Zusammensetzung (Mol-%) | Feed (Synthesegas (4) | CO-Produkt (zweites Retentat) (8) | H₂-Produkt (drittes Permeat) (11) | Rezyklat (14) |
|---|---|---|---|---|
| CO | 49,7% | 97,73% | 0,45% | 39,43% |
| H₂ | 49,6% | 1,0% | 99,4% | 59,7% |
| N₂ | 0,5% | 1,0% | 0,00% | 0,03% |
| CH₄ | 0,10% | 0,20% | 0,00% | 0,32% |
| CO₂ | 0,10% | 0,09% | 0,11% | 0,49% |

### Beispiel 2: Auftrennung eines Synthesegases (H₂ : CO = ca. 50 : 50) an einer Membran mit der H₂/CO Selektivität von 30

Beispiel 2 wurde weitestgehend wie Beispiel 1 durchgeführt. Im Folgenden sind deshalb nur die Unterschiede zu Beispiel 1 notiert. In Beispiel 2 wurde im Vergleich zu Beispiel 1 eine Membran mit einer H₂/CO Selektivität von 30 verwendet. Dies ist durch eine Reduzierung der H₂ Permeanz von 200 auf 150 GPU in der Simulation umgesetzt. Alle anderen Permeanzen sind gleich zu Tabelle 1. Auch der vorbehandelte Synthesegasstrom ist identisch mit Beispiel 1. Die Membranfläche beträgt in der ersten Membrantrennstufe 1438 m², in der zweiten Membrantrennstufe 4568 m² und in der dritten Membrantrennstufe 2877 m². In diesem Beispiel hat die zweite Trennstufe somit die höchste Kapazität. Der Normvolumenstrom des Synthesegases beträgt im vorliegenden Beispiel 10000 Nm³/h und der Normvolumenstrom des Rezyklats beträgt 6000 Nm³/h. Somit ergibt sich ein Verhältnis von Synthesegas zu Rezyklatstrom von 1,67 : 1. Insgesamt wird ein Normvolumenstrom des CO-Produkts (zweites Retentat) von 5047 Nm³/h erhalten. Die CO-Ausbeute der Membrantrennung beträgt 99,35 % und das CO-Produkt (zweites Retentat) weist eine Reinheit von 97,76% auf. Die Zusammensetzungen der einzelnen Ströme sind in Tabelle 3 gezeigt:

**Tabelle 3: Zusammensetzung der jeweiligen Ströme in Beispiel 2 / in Klammern sind die jeweiligen Bezugszeichen aus Fig. 1 angegeben.**

| Zusammensetzung (Mol-%) | Feed (Synthesegas (4) | CO-Produkt (zweites Retentat) (8) | H₂-Produkt (drittes Permeat) (11) | Rezyklat (14) |
|---|---|---|---|---|
| CO | 49,7% | 97,76% | 0,66% | 39,73% |
| H₂ | 49,6% | 1,0% | 99,2% | 59,5% |
| N₂ | 0,5% | 1,0% | 0,00% | 0,03% |
| CH₄ | 0,10% | 0,20% | 0,00% | 0,32% |
| CO₂ | 0,10% | 0,06% | 0,14% | 0,42% |

### Beispiel 3: Auftrennung eines Synthesegases (H₂ : CO = ca. 50 : 50) an einer Membran mit der H₂/CO Selektivität von 25

Beispiel 3 wurde weitestgehend wie Beispiel 1 durchgeführt. Im Folgenden sind deshalb nur die Unterschiede zu Beispiel 1 notiert. In Beispiel 3 wurde im Vergleich zu Beispiel 1 eine Membran mit einer H₂/CO Selektivität von 25 verwendet. Dies ist durch eine Reduzierung der H₂ Permeanz von 200 auf 125 GPU in der Simulation umgesetzt. Alle anderen Permeanzen sind gleich zu Tabelle 1. Auch der vorbehandelte Synthesegasstrom ist identisch mit Beispiel 1. Die Membranfläche beträgt in der ersten Membrantrennstufe 1729 m², in der zweiten Membrantrennstufe 5239 m² und in der dritten Membrantrennstufe 3458 m². In diesem Beispiel hat die zweite Trennstufe somit die höchste Kapazität. Der Normvolumenstrom des Synthesegases beträgt im vorliegenden Beispiel 10000 Nm³/h und der Normvolumenstrom des Rezyklats beträgt 7000 Nm³/h. Somit ergibt sich ein Verhältnis von Synthesegas zu Rezyklatstrom von 1,43 : 1. Insgesamt wird ein Normvolumenstrom des CO-Produkts (zweites Retentat) von 5039 Nm³/h erhalten. Die CO-Ausbeute der Membrantrennung beträgt 99,21 % und das CO-Produkt (zweites Retentat) weist eine Reinheit von 97,77% auf. Die Zusammensetzungen der einzelnen Ströme sind in Tabelle 4 gezeigt:

**Tabelle 4: Zusammensetzung der jeweiligen Ströme in Beispiel 3 / in Klammern sind die jeweiligen Bezugszeichen aus Fig. 1 angegeben.**

| Zusammensetzung (Mol-%) | Feed (Synthesegas (4) | CO-Produkt (zweites Retentat) (8) | H₂-Produkt (drittes Permeat) (11) | Rezyklat (14) |
|---|---|---|---|---|
| CO | 49,7% | 97,77% | 0,80% | 38,97% |
| H₂ | 49,6% | 1,0% | 99,0% | 60,3% |
| N₂ | 0,5% | 1,0% | 0,00% | 0,03% |
| CH₄ | 0,10% | 0,20% | 0,00% | 0,31% |
| CO₂ | 0,10% | 0,04% | 0,16% | 0,37% |

### Beispiel 4: Auftrennung eines Synthesegases (H₂ : CO = ca. 60 : 40) an einer Membran mit der H₂/CO Selektivität von 40

Beispiel 4 wurde weitestgehend wie Beispiel 1 durchgeführt. Im Folgenden sind deshalb nur die Unterschiede zu Beispiel 1 notiert. Die Membranfläche beträgt in der ersten Membrantrennstufe 1268 m², in der zweiten Membrantrennstufe 2901 m² und in der dritten Membrantrennstufe 2536 m². In diesem Beispiel hat die zweite Trennstufe somit die höchste Kapazität. Der Normvolumenstrom des Synthesegases beträgt im vorliegenden Beispiel 10000 Nm³/h und der Normvolumenstrom des Rezyklats beträgt 4000 Nm³/h. Somit ergibt sich ein Verhältnis von Synthesegas zu Rezyklatstrom von 2,5 : 1. Insgesamt wird ein Normvolumenstrom des CO-Produkts (zweites Retentat) von 4046 Nm³/h erhalten. Die CO-Ausbeute der Membrantrennung beträgt 99,37 % und das CO-Produkt (zweites Retentat) weist eine Reinheit von 97,43% auf. Die Zusammensetzungen der einzelnen Ströme sind in Tabelle 5 gezeigt:

**Tabelle 5: Zusammensetzung der jeweiligen Ströme in Beispiel 4 / in Klammern sind die jeweiligen Bezugszeichen aus Fig. 1 angegeben.**

| Zusammensetzung (Mol-%) | Feed (Synthesegas (4) | CO-Produkt (zweites Retentat) (8) | H₂-Produkt (drittes Permeat) (11) | Rezyklat (14) |
|---|---|---|---|---|
| CO | 39,7% | 97,43% | 0,42% | 40,93% |
| H₂ | 59,6% | 1,0% | 99,5% | 58,1% |
| N₂ | 0,5% | 1,2% | 0,00% | 0,04% |
| CH₄ | 0,10% | 0,25% | 0,00% | 0,41% |
| CO₂ | 0,10% | 0,09% | 0,11% | 0,53% |

### Beispiel 5: Auftrennung eines Synthesegases (H₂ : CO = ca. 40 : 60) an einer Membran mit der H₂/CO Selektivität von 40

Beispiel 5 wurde weitestgehend wie Beispiel 1 durchgeführt. Im Folgenden sind deshalb nur die Unterschiede zu Beispiel 1 notiert. Die Membranfläche beträgt in der ersten Membrantrennstufe 863 m², in der zweiten Membrantrennstufe 4708 m² und in der dritten Membrantrennstufe 1727 m². In diesem Beispiel hat die zweite Trennstufe somit die höchste Kapazität. Der Normvolumenstrom des Synthesegases beträgt im vorliegenden Beispiel 10000 Nm³/h und der Normvolumenstrom des Rezyklats beträgt 6000 Nm³/h. Somit ergibt sich ein Verhältnis von Synthesegas zu Rezyklatstrom von 1,67 : 1. Insgesamt wird ein Normvolumenstrom des CO-Produkts (zweites Retentat) von 6072 Nm3/h erhalten. Die CO-Ausbeute der Membrantrennung beträgt 99,66 % und das CO-Produkt (zweites Retentat) weist eine Reinheit von 97,93% auf. Die Zusammensetzungen der einzelnen Ströme sind in Tabelle 6 gezeigt:

**Tabelle 6: Zusammensetzung der jeweiligen Ströme in Beispiel 5 / in Klammern sind die jeweiligen Bezugszeichen aus Fig. 1 angegeben.**

| Zusammensetzung (Mol-%) | Feed (Synthesegas (4) | CO-Produkt (zweites Retentat) (8) | H₂-Produkt (drittes Permeat) (11) | Rezyklat (14) |
|---|---|---|---|---|
| CO | 59,7% | 97,93% | 0,51% | 38,30% |
| H₂ | 39,6% | 1,0% | 99,4% | 61,0% |
| N₂ | 0,5% | 0,8% | 0,00% | 0,03% |
| CH₄ | 0,10% | 0,16% | 0,00% | 0,26% |
| CO₂ | 0,10% | 0,09% | 0,12% | 0,46% |

### Beispiel 6: Auftrennung eines Synthesegases (H₂ : CO = ca. 50 : 50) an einer Membran mit der H₂/CO Selektivität von 40 und geringerer Rezirkulation

Beispiel 6 wurde weitestgehend wie Beispiel 1 durchgeführt. Im Folgenden sind deshalb nur die Unterschiede zu Beispiel 1 notiert. Die Membranfläche beträgt in der ersten Membrantrennstufe 1615 m², in der zweiten Membrantrennstufe 3201 m² und in der dritten Membrantrennstufe 3230 m². In diesem Beispiel hat die dritte Trennstufe somit die höchste Kapazität. Der Normvolumenstrom des Synthesegases beträgt im vorliegenden Beispiel 10000 Nm³/h und der Normvolumenstrom des Rezyklats beträgt 3000 Nm³/h. Somit ergibt sich ein Verhältnis von Synthesegas zu Rezyklatstrom von 3,33 : 1. Insgesamt wird ein Normvolumenstrom des CO-Produkts (zweites Retentat) von 4981 Nm³/h erhalten. Die CO-Ausbeute der Membrantrennung beträgt 98,05 % und das CO-Produkt (zweites Retentat) weist eine Reinheit von 97,76% auf. Die Zusammensetzungen der einzelnen Ströme sind in Tabelle 7 gezeigt:

**Tabelle 7: Zusammensetzung der jeweiligen Ströme in Beispiel 6 / in Klammern sind die jeweiligen Bezugszeichen aus Fig. 1 angegeben.**

| Zusammensetzung (Mol-%) | Feed (Synthesegas (4) | CO-Produkt (zweites Retentat) (8) | H₂-Produkt (drittes Permeat) (11) | Rezyklat (14) |
|---|---|---|---|---|
| CO | 49,7% | 97,76% | 1,93% | 63,91% |
| H₂ | 49,6% | 1,0% | 97,9% | 35,2% |
| N₂ | 0,5% | 1,0% | 0,01% | 0,05% |
| CH₄ | 0,10% | 0,20% | 0,00% | 0,52% |
| CO₂ | 0,10% | 0,05% | 0,15% | 0,34% |

Vergleicht man alle Simulationen miteinander (s. Tabellen 8), kann man erkennen, dass mit unterschiedlichen Strömen und unterschiedlichen Selektivitäten der Membranen sehr gute Ergebnisse erzielt werden konnten. Es konnten sehr reine Kohlenmonoxid-haltige Gasströme als zweites Retentat erhalten werden. Die CO-Ausbeute war zudem in allen Fällen sehr hoch.

**Tabelle 8: Übersicht der Versuchsdaten und Versuchsergebnisse aller Beispiele**

| Beispiel | Selektivität H₂/CO | Verhältnis H₂ : CO | Verhältnis Synthesegas : Rezyklatstrom | Reinheit zweites Retentat (CO-Gehalt) | Ausbeute CO |
|---|---|---|---|---|---|
| 1 | 40 | 50 : 50 | 2 : 1 | 97,73% | 99,55 % |
| 2 | 30 | 50 : 50 | 1,67 : 1 | 97,76% | 99,35 % |
| 3 | 25 | 50 : 50 | 1,43 : 1 | 97,77% | 99,21 % |
| 4 | 40 | 60 : 40 | 2,5 : 1 | 97,43% | 99,37 % |
| 5 | 40 | 40 : 60 | 1,67 : 1 | 97,93% | 99,66 % |
| 6 | 40 | 50 : 50 | 3,33 : 1 | 97,76% | 98,05 % |

## Patentansprüche

1. Verfahren zur Auftrennung von Synthesegas in einen Wasserstoff-reichen Gasstrom und einen Kohlenmonoxid-reichen Gasstrom in einer Membrantrenneinheit, welche mindestens drei Membrantrennstufen umfasst, wobei im Synthesegas ein Verhältnis von Wasserstoff zu Kohlenmonoxid im Bereich von 70 : 30 bis 30 : 70, bezogen auf den jeweiligen Volumenanteil von Wasserstoff und Kohlenmonoxid im Synthesegas, vorliegt, und wobei das Verfahren die folgenden Schritte aufweist:
a. Bereitstellen des Synthesegases und Vorbehandeln des Synthesegases zur zumindest teilweisen Abtrennung von einer oder mehreren im Synthesegas vorhandenen Nebenkomponente(n);
b. Zuführen eines Feedstroms, der das vorbehandelte Synthesegas und einen Rezyklatstrom umfasst, zur ersten Trennstufe unter Erhalt eines ersten Retentats und eines ersten Permeats, wobei Kohlenmonoxid im ersten Retentat und Wasserstoff im ersten Permeat angereichert wird;
c. Zuführen des ersten Retentats zur zweiten Trennstufe unter Erhalt eines zweiten Retentats und eines zweiten Permeats, wobei das zweite Rententat als Kohlenmonoxid-reicher Gasstrom entnommen wird und zu 85 Vol.-% oder mehr aus Kohlenmonoxid besteht und wobei das zweite Permeat vor die erste Trennstufe zurückgeführt wird;
d. Zuführen des ersten Permeats zur dritten Trennstufe unter Erhalt eines dritten Retentats und eines dritten Permeats, wobei das dritte Permeat als Wasserstoff-reicher Gasstrom entnommen wird und zu 70 Vol.-% oder mehr aus Wasserstoff besteht und wobei das dritte Retentat vor die erste Trennstufe zurückgeführt wird,
wobei das zweite Permeat und das dritte Rententat zu einem einzigen Rezyklatstrom vereint werden und der Druck des Rezyklatstroms mithilfe eines Kompressor erhöht wird, bevor der Rezyklatstrom vor der ersten Trennstufe mit dem vorbehandelten Synthesegas zu dem in Schritt b) eingesetzten Feedstrom kombiniert wird, wobei das Verhältnis von vorbehandeltem Synthesegas zu Rezyklatstrom im Feedstrom im Bereich 4 : 1 bis 1 : 1,5, bevorzugt 3 : 1 bis 1 : 1,2, besonders bevorzugt 2,5 : 1 bis 1 : 1 bezogen auf den jeweiligen Normvolumenstrom von vorbehandeltem Synthesegas und Rezyklatstrom ist.

2. Verfahren nach Anspruch 1, wobei das zweite Retentat zu 87 Vol.-% oder mehr, vorzugsweise zu 91 Vol.-%, besonders bevorzugt zu 95 Vol.-% oder mehr aus Kohlenmonoxid besteht.

3. Verfahren nach Anspruch 1 oder 2, wobei das zweite Rententat höchstens 2 Vol.-%, vorzugsweise höchstens 1 Vol.-% an Wasserstoff enthält.

4. Verfahren nach einem der vorhergehenden Ansprüche, wobei das dritte Permeat in Abhängigkeit von der Zusammensetzung des Synthesegases neben dem Wasserstoff aus weiteren gasförmigen Stoffen wie Kohlendioxid, Kohlenmonoxid besteht.

5. Verfahren nach Anspruch 4, wobei das dritte Permeat weniger als 3 Vol.-%, vorzugsweise weniger als 2 Vol.-%, besonders bevorzugt weniger als 1 Vol.-% an Kohlenmonoxid enthält.

6. Verfahren nach einem der vorhergehenden Ansprüche, wobei die zweite Trennstufe oder die dritte Trennstufe, vorzugsweise die zweite Trennstufe die höchste Kapazität aufweist.

7. Verfahren nach einem der vorhergehenden Ansprüche, wobei der permeatseitige Druck der ersten Trennstufe zwischen 2,5 und 30 bar, vorzugsweise mindestens zwischen 3,0 und 26 bar, besonders bevorzugt zwischen mindestens 3,3 und 21 bar beträgt.

8. Verfahren nach einem der vorhergehenden Ansprüche, wobei der retentatseitige Druck der ersten Trennstufe mindestens 20 bis 80 bar, vorzugsweise 25 bis 65 bar, besonders bevorzugt 30 bis 45 beträgt.

9. Verfahren nach einem der vorhergehenden Ansprüche, wobei der Transmembrandruck in der ersten Trennstufe 9 bis 75 bar, vorzugsweise 9 bis 60 bar, besonders bevorzugt 13 bis 40 bar beträgt.

10. Verfahren nach einem der vorhergehenden Ansprüche, wobei der permeatseitige Druck der zweiten Trennstufe zwischen 2,0 und 30 bar, vorzugsweise mindestens zwischen 2,5 und 26 bar, besonders bevorzugt zwischen mindestens 2,8 und 21 bar beträgt.

11. Verfahren nach einem der vorhergehenden Ansprüche, wobei bei dem Verfahren in allen drei Trennstufen eine Temperatur von 15 bis 100 °C, vorzugsweise zwischen 25 und 60 °C vorliegt.

12. Verfahren nach einem der vorhergehenden Ansprüche, wobei die drei Trennstufen jeweils aus einem oder mehreren Trennmodulen bestehen, wobei bei Vorliegen von mehreren Trennmodulen, diese Trennmodule innerhalb einer Trennstufe parallel und/oder seriell verschaltet sind.

13. Verfahren nach Anspruch 12, wobei die Trennmodule eine Reinstoffselektivität bei 25 °C für Wasserstoff/Kohlenmonoxid im Bereich von mindestens 25, vorzugsweise mindestens 30, besonders bevorzugt mindestens 40 aufweisen.

14. Verfahren nach einem der vorhergehenden Ansprüche, wobei als Membranmaterial ein Material, ausgewählt aus der Gruppe, bestehend aus Polyimiden, Polyamiden, Polysulfonen, Celluloseacetaten und deren Derivaten, Polyphenylenoxiden, Polysiloxanen, Polymeren mit intrinsischer Mikroporosität, Mixed Matrix Membranen, Facilitated Transport Membranen, Polyethylenoxiden, Polypropylenoxiden, Kohlenstoffmembranen, Zeolithe und Mischungen daraus, eingesetzt wird.

15. Integriertes Verfahren zur Alkoxycarbonylierung von C2- bis C20-Kohlenwasserstoffen mit mindestens einer olefinischen Doppelbindung mit einem Kohlenmonoxid-Strom und mit einem Alkohol in Gegenwart eines homogenen Katalysatorsystems in einer Reaktionszone, wobei das in Schritt b) des Verfahrens nach einem der Ansprüche 1 bis 14 bereitgestellte zweite Retentat als Kohlenmonoxid-Strom eingesetzt wird.
